(19) Europäisches Patentamt
European Patent Office
Office européen des brevets

(11) Veröffentlichungsnummer: **0 355 024 B1**

(12) **EUROPÄISCHE PATENTSCHRIFT**

(45) Veröffentlichungstag der Patentschrift: **15.09.93**

(51) Int. Cl.5: **C07D 235/26**

(21) Anmeldenummer: **89114993.2**

(22) Anmeldetag: **14.08.89**

# (54) Verfahren zur Herstellung von cyclischen Harnstoffen.

(30) Priorität: **17.08.88 DE 3827867**

(43) Veröffentlichungstag der Anmeldung:
**21.02.90 Patentblatt 90/08**

(45) Bekanntmachung des Hinweises auf die Patenterteilung:
**15.09.93 Patentblatt 93/37**

(84) Benannte Vertragsstaaten:
**CH DE FR GB IT LI**

(56) Entgegenhaltungen:
**EP-A- 0 013 859**
**DE-A- 3 124 618**
**US-A- 4 282 193**
**US-A- 4 282 194**

**CHEMICAL ABSTRACTS, Band 107, Nr. 5, 3. August 1987, Columbus, Ohio, USA; NOMURA R. et al.: "Preparation of cyclic ureas carbon dioxide and diamines catalyzedby triphenyl stibine oxide", Seite 688, Spalte 1, Zusammenfassung-Nr. 39 679f**

(73) Patentinhaber: **HOECHST AKTIENGESELLSCHAFT**

**D-65926 Frankfurt(DE)**

(72) Erfinder: **Fischer, Hartmut, Dr.**
**Wilhelm-Leuschner-Strasse 5**
**D-6238 Hofheim am Taunus(DE)**

EP 0 355 024 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf die Herstellung von cyclischen Harnstoffen. Diese werden heute im technischen Maßstab durch Umsetzung der entsprechenden Diaminoaromaten, im einfachsten Fall des o-Phenylendiamins, mit Kohlensäurederivaten wie Harnstoff oder Phosgen hergestellt. Nachteilig an diesem Verfahren ist der Zwangsanfall von erheblichen Mengen Ammoniumsalzen sowie bei Einsatz von Phosgen die Handhabung dieser toxischen Substanz.

Es hat nicht an Versuchen gefehlt, cyclische Harnstoffe aus Diaminoverbindungen und Kohlendioxyd direkt zu synthetisieren. Dabei gelang die Umsetzung trotz Anwendung hoher Drucke und Temperaturen nur unvollständig. Die Ausbeute konnte durch Zusatz von wasserbindenden Mitteln, wie Cyclohexylcarbodiimid, zwar verbessert werden, blieb jedoch unbefriedigend. Eine weitere Verbesserung wurde von R. Nomura, M. Yamamoto und H. Matsuda beschrieben (Ind. Eng. Chem. Res. 1987, 1056 - 1059), die im Triphenylantimonoxid einen wirksamen Katalysator für die Reaktion erkannten. Die katalytische Wirkung ist allerdings nur für die Umsetzung aliphatischer Diamine erwiesen, und zudem ist der Einsatz dieser metallorganischen Verbindung in einem technischen Prozeß nicht vorteilhaft, so daß dieser Weg nicht weiter verfolgt wurde.

Aus der DOS 1543748 (= US PS 3413350 und 3414619) ist bekannt, aromatische o-Diamine mit $CO_2$, vorzugsweise in Gegenwart von Alkali- oder Erdalkaliverbindungen umzusetzen, um die o-Diamine aus Gemischen zu entfernen, die überwiegend p-Isomere enthalten. Unter den basischen Alkali- und Erdalkaliverbindungen werden neben vielen anderen auch Natrium-, Kalium- und sogar Kalziumcarbonat genannt. In der Beschreibung der DOS 1543748 wird auch erwähnt, daß die anwesende Menge an o-Diamin in den Isomerengemischen keine Rolle spiele, daß geringfügige Mengen ebenso entfernt werden wie größere, ja sogar 100 % aromatische o-Diamine sich mit Kohlendioxyd umsetzen ließen. Wie eigene Versuche ergeben haben, ist diese Reaktion jedoch für die Umsetzung reiner o-Diamine zu Benzimidazolonen nicht geeignet, weil sie nur sehr langsam abläuft und vor allem, weil das Reaktionsgemisch nach der Bildung größerer Mengen des cyclischen Harnstoffs wegen deren hohen Schmelzpunkten eine solche Konsistenz bekommt, daß es nicht mehr gerührt werden kann, ein Zustand, der bei Umsätzen zwischen 30 und 50 % eintritt. Der hohe Schmelzpunkt und die geringe Löslichkeit der Endprodukte erschweren also deswegen die praktische technische Durchführung, weil es schon bei niederen Umsetzungsgraden nicht mehr möglich ist, zu rühren und den Feststoff zu fördern und auszutragen.

Es war daher erwünscht, ein einfaches und störungsfreies Verfahren zur Umsetzung von ortho-Diaminen mit $CO_2$ zu entwickeln. Hierfür war es aber erforderlich, ein geeignetes flüssiges Reaktionsmedium zu finden, in dem einerseits die Umsetzung gut abläuft und das es andererseits erlaubt, das Produkt aus dem Reaktor auszuschleusen. Bei der Suche nach einem solchen geeigneten flüssigen Reaktionsmedium zeigte es sich, daß alle naheliegenden organischen Lösungsmittel wie Alkohole, Äther, Amine oder Amide keine geeigneten Lösemittel für das Produkt sind oder die Umsetzung inhibieren.

Es wurde nun überraschend gefunden, daß die Synthese von cyclischen Harnstoffen aus ortho-Diaminen und $CO_2$ in wäßrigen Systemen möglich ist und hierin sogar besonders gut gelingt.

Dieses Ergebnis überrascht um so mehr, als zu erwarten war, daß Wasser als Reaktionsprodukt der Harnstoffbildung die Synthese hemmen sollte. In einer Arbeit von T.V. Charlu und M.R.A. Rao (Indian J. Technol. 1969, 111 - 114) wird die Wirkung des Reaktionswassers für die Ausbeute bei der Synthese von Imidazolonen aus 1,2-Diaminen und $CO_2$ als verheerend ("detrimental") bezeichnet (s. Zusammenfassung. Dementsprechend sind aus der Literatur auch Methoden bekannt, um das störende Wasser zu entfernen, z. B. durch Zusatz von Carbodiimiden oder $PCl_3$.

Gegenstand der Erfindung ist ein Verfahren zur Herstellung der cyclischen Harnstoffe des Strukturtyps der Benzimidazolone I (s. Patentanspruch 1), worin $R^1$ und $R^2$ unabhängig voneinander Wasserstoff, Alkyl mit 1 bis 6 C-Atomen oder Alkoxy mit 1 bis 3 C-Atomen sein können, durch Umsetzung der entsprechenden Diaminoverbindung II (s. Patentanspruch 1) mit Kohlendioxyd, gegebenenfalls in Gegenwart von Katalysatoren, welches dadurch gekennzeichnet ist, daß die Umsetzung in Gegenwart von Wasser als Reaktionsmedium unter erhöhtem Druck bei einer Temperatur von mindestens 120° C durchgeführt wird. Der Einsatz von Wasser erweist sich als außerordentlich vorteilhaft, weil das Verfahren durch die permanente Anwesenheit des flüssigen Mediums Wasser auch bei hohen Umsätzen technisch beherrschbar bleibt. Es entstehen auch bei hohen Umsätzen praktisch keine Nebenprodukte. Die Trennung von Produkt, Reaktionsmedium, Katalysator und eventuellen geringen Resten an Ausgangsstoff (1,2-Diamin) gelingt durch einfache Filtration und Waschen mit Wasser; Wasser, Katalysator und restliches Ausgangsmaterial können beliebig oft in den Prozeß zurückgeführt werden, so daß die angestrebten Produkte der Formel I hergestellt werden können, ohne daß Entsorgungsprobleme auftreten.

Das Verfahren ist anwendbar auf Ausgangsstoffe der Struktur II, wobei $R^1$ und $R^2$ entweder Wasserstoffatome oder niedere Alkylreste wie Methyl, Äthyl oder die verschiedenen Propyl-, Butyl-, Pentyl- oder

Hexylreste, oder Alkoxyreste mit 1 bis 3 C-Atomen sein können, wobei vorzugsweise nur höchstens einer der Reste Alkoxy ist. Die Alkylreste enthalten vorteilhaft 1 bis 4, insbesondere 1 oder 2 C-Atome und die Alkoxyreste vorzugsweise 1 bis 2 C-Atome, insbesondere nur 1 C-Atom. Ganz besonders bevorzugte Ausgangsstoffe sind solche, in denen wenigstens eine der Reste $R^1$ und $R^2$ Wasserstoff ist, insbesondere 1,2-Diaminobenzol und die beiden 1,2-Diaminotoluole.

Das erfindungsgemäße Verfahren muß in einem Druckreaktor ausgeführt werden. Die Ausgangsstoffe können als wäßrige Lösung bei gewöhnlichem Druck vorgelegt oder auch unter Druck über eine Pumpe eingetragen werden. Das Kohlendioxyd wird zweckmäßig über eine Dosiervorrichtung unter Druck flüssig oder gasförmig eingegeben, wodurch es möglich wird, den Druckreaktor mit hoher Ausnutzung des Volumens und auch kontinuierlich zu betreiben.

Bezogen auf die Diaminoverbindug kann die eingesetzte Wassermenge in weiten Grenzen variiert werden. Beispielsweise verwendet man zwischen 20 und 800, z. B. bis 500 Gew.-% Wasser, bezogen auf die Diaminoverbindung. Bevorzugt wird mit Wassermengen zwischen 50 und 400, oft bis 300 Gew.-% gearbeitet. Damit werden bei erhöhter Temperatur homogene Lösungen der Ausgangsstoffen erreicht; es wird ein sehr hoher Umsatz von über 95 % erreicht, und als Produkt fällt eine gut rühr- und förderfähige wäßrige Suspension des cyclischen Harnstoffes an, was für die technische Ausführung des Verfahrens angesichts der geringen Löslichkeit und des hohen Schmelzpunktes der Verbindungen II sehr vorteilhaft ist.

Die Umsetzung der Diaminoverbindung mit dem $CO_2$ wird durch basisch reagierende wasserlösliche Verbindungen beschleunigt, bei deren Mitverwendung sich besonders hohe Umsätze erzielen lassen. Daher ist es vorteilhaft, dem Reaktionsmedium Wasser solche Basen zuzusetzen. Ihre Menge liegt im allgemeinen zwischen 2 und 100, oft bis 30 Mol-%, bezogen auf die Diaminoverbindung; bevorzugt werden 10 bis 50, oft bis 25 Mol-% des basischen Katalysators zugegeben. Als basische Katalysatoren sind vor allem basisch reagierende Alkalimetallverbindungen geeignet, z. B. die Carbonate, Hydrogencarbonate und Hydroxyde der Alkalimetalle, wie des Natriums, insbesondere des Kaliums, Ammoniumcarbonat und wäßrige Ammoniaklösung; ebenfalls geeignet sind organische Basen wie Trialkylamine, z. B. Triäthyl-, Tri-n- oder -iso-propyl- oder die verschiedenen Tributylamine oder Triamine mit verschiedenen Alkylresten, oder Morpholin. Ein besonders bevorzugt eingesetzter Katalysator ist Kaliumcarbonat, weil es besonders wirksam ist, aus dem Produkt gut auswaschbar und vollständig zurückgeführt werden kann.

Die Reaktionstemperatur liegt zweckmäßig zwischen 150 und 260° C. Steigende Reaktionstempertur beschleunigt die Umsetzung, übermäßig hohe Temperatur vermindert jedoch die Selektivität. Der optimale und am meisten bevorzugte Temperaturbereich liegt zwischen 200 und 240° C.

Das Kohlendioxyd kann am Anfang im großen Überschuß, z. B. der doppelt molaren Menge, eingesetzt werden. Man kann auch mit einem geringeren Überschuß oder gar mit weniger als der molaren Menge beginnen, wenn während der Reaktion das verbrauchte $CO_2$ laufend nachdosiert wird. Es ist zu beachten, daß sich der Gesamtdruck des Systems zusammensetzt aus dem Wasserdampfpartialdruck, der von der Temperatur abhängt, und dem $CO_2$-Partialdruck, der aus der eingebrachten bzw. noch vorhandenen $CO_2$-Menge resultiert. Der Mindestpartialdruck an $CO_2$ bei Reaktionstemperatur sollte 1 bar nicht unterschreiten. Der Partialdruck kann z. B. bis zu 200 bar betragen; zweckmäßig wird er zwischen 5 und 50 bar gehalten. Bevorzugt wird während der Reaktionsphase ein $CO_2$-Druck von 10 bis 30 bar aufrechterhalten. Im bevorzugten Temperaturbereich zwischen 200 und 240° C resultiert daraus ein Gesamtdruck von 25 bis 64 bar. Zweckmäßig sollte der Druckbehälter mindestens auf diesen Druck ausgelegt sein. Nach beendeter Reaktion wird auf eine Temperatur unter 100° C abgekühlt, wobei das erhaltene Gemisch zweckmäßig langsam gerührt wird, um den kristallisierenden cyclsichen Harnstoff feinteilig im wäßrigen Reaktionsmedium zu verteilen. In dieser Form kann das Produkt, nach Entspannen des überschüssigen Kohlendioxyds, aus dem Reaktor entnommen werden. Die Trennung von dem wäßrigen Reaktionsmedium, z. B. der Katalysatorlösung, gelingt sehr einfach durch Filtration. Die Flüssigkeit, die gegebenenfalls einen Katalysator zusammen mit Resten nicht umgesetzter Diaminoverbindung enthält, kann in den Prozeß zurückgeführt werden. Das Verfahren zeichnet sich daher durch eine deutlich verminderte Abwasserbelastung aus.

## Beispiele

1. In einem Druckreaktor von 200 ml Inhalt wurden 43,2 g (0,4 Mol 1,2-Diaminobenzol und eine Lösung von 11 g (0,08 Mol) Kaliumcarbonat in 54 g Wasser vorgelegt. Anschließend wurde der geschlossene Reaktor mit $CO_2$ bis zu einem Druck von 30 bar befüllt und mit einer elektrischen Heizung auf Reaktionstemperatur gebracht. Dabei stieg der Gesamtdruck zunächst an und erreichte in der Aufheizphase bei 180° C einen Maximalwert von 62 bar, der dann infolge der einsetzenden Reaktion wieder langsam abfiel. Dann wurde weiter aufgeheizt. Als sich bei der konstanten Reaktionstemperatur von 230° C ein Gesamtdruck von 40 bar eingestellt hatte, wurde dieser durch Nachpressen von $CO_2$ über 6 h

konstant gehalten. Danach wurde abgekühlt und der Rest $CO_2$ entspannt.

Aus dem geöffneten Druckreaktor wurde das in der Katalysatorlösung suspendierte Benzimidazolon entnommen und von dieser durch Filtration abgetrennt. (Nach Ergänzung der Verluste kann diese für den nächsten Ansatz in den Reaktor zurückgeführt werden.) Das Benzimidazolon wurde mit 50 ml Wasser auf dem Filter gewaschen und anschließend getrocknet. Das Material war gut kristallisiert, schwach graublau gefärbt und hatte einen Schmelzpunkt von 318° C.

Ausbeute: 48,9 g = 91,2 % d. Th.

2. 1080 g (10 Mol) 1,2-Diaminobenzol, 276 g (2 Mol $K_2CO_3$ und 1700 g Wasser wurden gemischt und durch Erwärmen auf 90° C zu einer homogenen Lösung gebracht, die in einen vorgewärmten Druckreaktor von 10 l Volumen gegeben wurde. Danach wurden 1000 g (23 Mol) $CO_2$ eingepumpt und die Temperatur auf 230° C erhöht und über 6 h gehalten. Der Druck stieg während der Aufheizzeit auf maximal 55 bar. Nach beendeter Umsetzung wurde abgekühlt, das überschüssige $CO_2$ entspannt und das Reaktionsprodukt entnommen. Das gebildete Benzimidazolon wurde in einem Druckfilter von der den Katalysator enthaltenden Lösung getrennt und der Filterkuchen mit 300 g Wasser gewaschen, um die noch anhaftenden Reste der Katalysatorlösung vom Produkt zu verdrängen. Die zurückgewonnene Katalysatorlösung, die noch wenig nicht umgesetztes 1,2-Diaminobenzol enthielt, wurde in den Prozeß zurückgeführt. Im hier beschriebenen Beispiel wurde die Katalysatorlösung zweimal zurückgeführt; die Einzelergebnisse sind in der Tabelle zusammengefaßt.

**3.** In einen 200 ml-Reaktor wurden 24,4 g (0,2 Mol) 3,4-Diaminotoluol, 18 g Wasser und 5,5 g (0,04 Mol) Kaliumcarbonat gefüllt. Nach dem Verschließen wurde $CO_2$ bis zu einem Druck von 35 bar aufgedrückt. Anschließend wurde auf 230° C erwärmt und diese Temperatur 12 Stunden gehalten. Während des Aufheizens stieg der Druck auf bis zu 80 bar. Nach beendeter Reaktion wurde abgekühlt, das überschüssige $CO_2$ entspannt und der wäßrige Kristallbrei entnommen. Über ein Filter wurde die wäßrige Katalysatorlösung abgetrennt, der Feststoff mit Wasser gewaschen, bis er kein Carbonat mehr enthielt, und getrocknet. Es wurden 27,7 g (93,7 % d.Th.) 5-Methyl-benzimidazolon von Fp = 301 - 303° C isoliert.

**4.** Das Beispiel 3 wurde wiederholt, wobei anstelle des 3,4-Diaminotoluols jedoch 27,3 g (0,2 Mol 4,5-Diamino-ortho-xylol eingesetzt wurden. Nach Aufarbeitung des Ansatzes wurden 31,3 g (96 % d.Th) 5,6-Dimethylbenzimidazolon ($F_p$ über 360° C) isoliert.

**5.** Das Beispiel 3 wurde wiederholt, wobei als Ausgangsstoff 27,6 g (0,2 Mol) 3,4-Diaminoanisol eingesetzt wurden. Als Produkt wurden 30,5 g (93,0 % d.Th.) 5-Methoxybenzimidazolon vom $F_p$ 256° C isoliert.

**6.** In einen Druckreaktor von 200 ml Inhalt wurden 43,2 g (0,4 Mol) 1,2-Diaminobenzol, 54 g Wasser und 7,7 ml einer konzentrierten wäßrigen Ammoniaklösung (entsprechend 0,1 Mol $NH_3$) eingefüllt. Anschließend wurde der Reaktor mit $CO_2$ bis zu einem Druck von 40 bar beschickt. Während der Aufheizphase stieg der Druck bis maximal 80 bar an und fiel nach Erreichen der Reaktionstemperatur von 210° C langsam wieder ab. Durch Nachpressen von $CO_2$ wurde über 12 Stunden ein konstanter Druck von 50 bar eingestellt. Danach wurde abgekühlt und der Rest des $CO_2$ entspannt. Das Produkt wurde entnommen, von der Katalysatorlösung abfiltriert, mit 50 ml Wasser gewaschen und getrocknet. Die Ausbeute an Benzimidazolon betrug 46,6 g = 87,0 % d. Th. Die Tatsache, daß die Ausbeute geringer ist als in Beispielen 1 bis 5, ist deswegen ohne große praktische Bedeutung, weil unumgesetztes Diamin und das wäßrige Reaktionsmedium nach Abtrennung des gewünschten Produkts in den Produktionskreislauf zurückgeführt werden können.

**7. und 8.** Das Beispiel 6 wurde wiederholt, jedoch wurden anstelle der wäßrigen Ammoniaklösung 8,7 g (0,1 Mol) Morpholin als Katalysator eingesetzt. Nach Aufarbeitung wurden 41,9 g = 78,2 % d. Th. Benzimidazolon isoliert. Wurden als Katalysator 10,1 g (0,1 Mol) Triäthylamin eingesetzt, so erhielt man ein ähnliches Ergebnis.

Tabelle zu Beispiel 2 a - c

| | Wasser | $K_2CO_3$ | Einsatz $Ph(NH_2)_2$ | Filtrat + 1. Waschwasser | Rest $Ph(NH_2)_2$ | | aus Versuch | Benzimidazolon isoliert, getrocknet | |
|---|---|---|---|---|---|---|---|---|---|
| | g | g | g | g | g | % | | | |
| a | 1700 | 276 | 1080 | | | | | 1250 | 93,3 |
| b | | | 1125 | 1980 | 18 | = 1,7 | a | 1298 | 91,5 |
| c | | | 1136 | 1980 | 35 | = 3,2 | b | 1328 | 92,2 |
| | | | | 1980 | 37 | = 3,3 | c | | |

9. Das Beispiel 1 wurde wiederholt, wobei jedoch das molare Verhältnis von Wasser und Alkalicarbonat zur Diaminoverbindung erhöht wurde.
In einem Druckreaktor von 200 ml Inhalt wurden 21,6 g (0,2 Mol) 1,2-Diaminobenzol, 108 g Wasser und 27,6 g (0,2 Mol) Kaliumcarbonat eingefüllt. Der Reaktor wurde verschlossen und bei ca. 50° C Innentemperatur mit $CO_2$ gefüllt bis zu einem Druck von 35 bar. Anschließend wurde auf Reaktionstemperatur von 230° C geheizt, wobei der Druck kurzzeitig 65 bar erreichte, mit Einsetzen der Reaktion aber langsam abfiel. Durch Nachdrücken von $CO_2$ wurden 58 bis 60 bar über 6 h gehalten. Danach wurde abgekühlt auf Zimmertemperatur und das überflüssige $CO_2$ abgeblasen. Aus dem drucklosen Reaktor wurde die Produktsuspension entnommen und das Benzimidazolon durch Filtration isoliert. Nach Waschen und Trocknung wurden 24,0 g (89,6 % d.Th.) Benzimidazolon erhalten.

**10**. Wie in Beispiel 9 wurden 21,6 g (0,2 Mol) 1,2-Diaminobenzol in 108 g Wasser eingesetzt, als $CO_2$-Überträger wurden jedoch 21,2 g (0,2 Mol) $Na_2CO_3$ zugegeben. Die Umsetzung mit $CO_2$ wurde bei 210° C über 6 h bei einem Gesamtdruck von 58 bis 62 bar durchgeführt. Nach Beendigung der Reaktion (Druckkonstanz) wurde auf 130° C abgekühlt und bei dieser Temperatur das überschüssige $CO_2$ entspannt. Die Entspannung wurde fortgesetzt, bis die Gasphase des Systems weitgehend aus Wasserdampf bestand. Durch diese Maßnahme wurde die Auskristallisation von Natriumhydrogencarbonat bei Abkühlung auf Zimmertemperatur verhindert und in der Natriumcarbonatlösung war nur das fast farblose, feinkristalline Benzimidazolon suspendiert, das durch Filtration isoliert wurde.
Ausbeute: 24,4 g (91,0% d.Th.).

**11.** Es wurde analog zu Beispiel 10 gearbeitet, aber als Reaktionsmedium wurde eine kleinere Menge einer geringer konzentrierten Natriumcarbonatlösung eingesetzt. In den Reaktor wurden 21,6 g (0,2 Mol) 1,2-Diaminobenzol, 90 g Wasser und 10,6 g (0,1 Mol) Natriumcarbonat eingefüllt. Die Umsetzung mit $CO_2$ wurde bei 210° C über 6 h bei einem Gesamtdruck von 58 bis 62 bar durchgeführt. Nach Abblasen des $CO_2$ bei 130° C wurde weiter auf Zimmertemperatur abgekühlt und das Benzimidazolon isoliert.
Ausbeute: 22,5 g (84,0% d.Th.)

## Patentansprüche

1. Verfahren zur Herstellung von cyclischen Harnstoffen der Formel

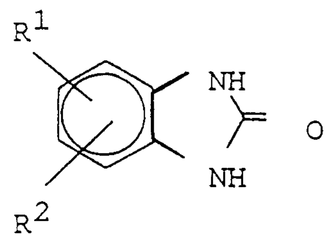

(I),

in der $R^1$ und $R^2$ Wasserstoff, Alkyl mit 1 bis 6 C-Atomen und/oder Alkoxy mit 1 bis 3 C-Atomen sind, durch Umsetzung von 1,2-Diaminobenzolen der Formel

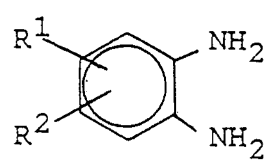

(II),

in der $R^1$ und $R^2$ die angegebene Bedeutung haben, mit Kohlendioxyd in Gegenwart oder Abwesenheit von basischen Verbindungen, dadurch gekennzeichnet, daß man die Umsetzung in Gegenwart von Wasser als Reaktionsmedium unter erhöhtem Druck bei einer Temperatur von mindestens 120° C durchführt.

2. Verfahren nach Anspruch 1, dadurch gekennzeichnet, daß die angewandte Wassermenge zwischen 20 und 800, bevorzugt zwischen 50 und 400, insbesondere zwischen 50 und 300 Gew., %, bezogen auf die Diaminoverbindung-variiert.

3. Verfahren nach Anspruch 1 oder 2, dadurch gekennzeichnet, daß die Umsetzung in Gegenwart von 2 bis 100 Mol-%, vorzugsweise von 10 bis 50, insbesondere von 10 bis 25 Mol-% eines basischen Katalysators, insbesondere von wasserlöslichen, basisch reagierenden Alkalimetallverbindungen, insbesondere Kaliumcarbonat, durchgeführt wird.

**4.** Verfahren nach einem oder mehreren der Ansprüche 1 bis 3, dadurch gekennzeichnet, daß die Umsetzung bei einer Temperatur von 150 bis 260 ° C, vorzugsweise von 200 bis 240 ° C durchgeführt wird.

**5.** Verfahren nach einem oder mehreren der Ansprüche 1 bis 4, dadurch gekennzeichnet, daß das Kohlendioxid zu Beginn der Reaktion in mindestens der doppelt molaren Menge eingesetzt wird.

**6.** Verfahren nach einem oder mehreren der Ansprüche 1 bis 5, dadurch gekennzeichnet, daß die Umsetzung bei einem Kohlendioxyd-Partialdruck von mindestens 1 und bis 200 bar, vorzugsweise von 5 bis 50 bar und insbesondere von 10 bis 30 bar durchgeführt wird.

**7.** Verfahren nach einem oder mehreren der Ansprüche 1 bis 6, dadurch gekennzeichnet, daß während der Reaktion laufend Kohlendioxyd unter Druck zugeführt wird.

**8.** Verfahren nach einem oder mehreren der Ansprüche 1 bis 7, dadurch gekennzeichnet, daß Verbindungen der Formel II umgesetzt werden, in denen höchstens einer der Reste $R^1$ und $R^2$ Alkoxy und vorzugsweise wenigstens einer dieser Reste Wasserstoff ist.

**9.** Verfahren nach einem oder mehreren der Ansprüche 1 bis 8, dadurch gekennzeichnet, daß die Alkylreste 1 bis 4, insbesondere 1 bis 2 C-Atome enthalten und die Alkoxyreste 1 bis 2 C-Atome, insbesondere nur 1 C-Atom.

**10.** Verfahren nach einem oder mehreren der Ansprüche 1 bis 9, dadurch gekennzeichnet, daß der cyclische Harnstoff von dem wäßrigen Reaktionsmedium durch Filtration abgetrennt wird und die Flüssigkeit, die gegebenenfalls einen Katalysator und Resten nicht umgesetzten Ausgangsmaterials enthält, in das Verfahren zurückgeführt wird.

## Claims

**1.** A process for the preparation of cyclic ureas of the formula

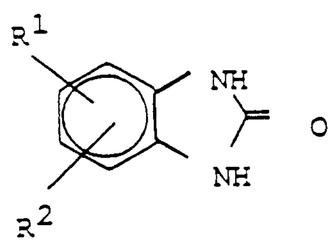

(I),

in which $R^1$ and $R^2$ are hydrogen, alkyl having 1 to 6 carbon atoms and/or alkoxy having 1 to 3 carbon atoms, by reaction of 1,2-diaminobenzenes of the formula

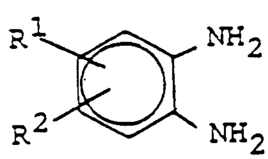

(II),

in which $R^1$ and $R^2$ have the meaning given, with carbon dioxide in the presence or absence of basic compounds, which comprises carrying out the reaction in the presence of water as the reaction medium under elevated pressure at a temperature of at least 120 ° C.

2. The process as claimed in claim 1, wherein the amount of water used varies between 20 and 800, preferably between 50 and 400, in particular between 50 and 300, % by weight, relative to the diamino compound.

3. The process as claimed in claim 1 or 2, wherein the reaction is carried out in the presence of 2 to 100 mol %, preferably 10 to 50, in particular 10 to 25, mol % of a basic catalyst, in particular of water-soluble, basic alkali metal compounds, in particular potassium carbonate.

4. The process as claimed in one or more of claims 1 to 3, wherein the reaction is carried out at a temperature of 150 to 260 °C, preferably 200 to 240 °C.

5. The process as claimed in one or more of claims 1 to 4, wherein the carbon dioxide is used at the beginning of the reaction in at least twice the molar amount.

6. The process as claimed in one or more of claims 1 to 5, wherein the reaction is carried out at a carbon dioxide partial pressure of at least 1 and up to 200 bar, preferably 5 to 50 bar, and in particular 10 to 30 bar.

7. The process as claimed in one or more of claims 1 to 6, wherein carbon dioxide is continuously added under pressure during the reaction.

8. The process as claimed in one or more of claims 1 to 7, wherein compounds of the formula II are reacted in which at most one of the radicals $R^1$ and $R^2$ is alkoxy and preferably at least one of these radicals is hydrogen.

9. The process as claimed in one or more of claims 1 to 8, wherein the alkyl radicals contain 1 to 4, in particular 1 to 2, carbon atoms, and the alkoxy radicals contain 1 to 2 carbon atoms, in particular only 1 carbon atom.

10. The process as claimed in one or more of claims 1 to 9, wherein the cyclic urea is separated off from the aqueous reaction medium by filtration, and the liquid, which may contain a catalyst and residual unconverted starting material, is recycled into the process.

## Revendications

1. Procédé pour préparer des urées cycliques de formule

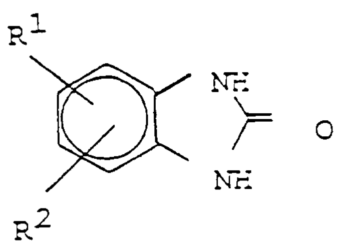

(I),

[dans laquelle $R^1$ et $R^2$ représentent chacun un atome d'hydrogène, un groupe alkyle ayant 1 à 6 atomes et/ou un groupe alcoxy ayant 1 à 3 atomes de carbone], par réaction de 1,2-diaminobenzènes de formule :

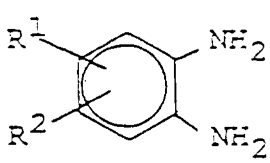

(II),

[dans laquelle $R^1$ et $R^2$ ont le sens indiqué] avec du bioxyde de carbone en présence ou en l'absence de composés basiques, procédé caractérisé en ce qu'on conduit la réaction en présence d'eau comme milieu de réaction, sous pression élevée, à une température d'au moins 120 °C.

**2.** Procédé selon la revendication 1, caractérisé en ce qu'on fait varier entre 20 et 800, de préférence entre 50 et 400, notamment entre 50 et 300 % en poids, par rapport à la diamine, la quantité d'eau utilisée.

**3.** Procédé selon la revendication 1 ou 2, caractérisé en ce qu'on conduit la réaction en présence de 2 à 100 moles %, avantageusement de 10 à 50, notamment de 10 à 25 moles % d'un catalyseur basique, notamment de composés de métaux alcalins hydrosolubles à réaction basique, notamment le carbonate de potassium.

**4.** Procédé selon une ou plusieurs des revendications 1 à 3, caractérisé en ce qu'on conduit la réaction à une température de 150 à 260 °C, avantageusement de 200 à 240 °C.

**5.** Procédé selon une ou plusieurs des revendications 1 à 4, caractérisé en ce qu'on introduit au début de la réaction le bioxyde de carbone en une quantité molaire au moins double.

**6.** Procédé selon une ou plusieurs des revendications 1 à 5, caractérisé en ce qu'on conduit la réaction sous une pression partielle du bioxyde de carbone valant au moins 1 et allant jusqu'à 200 bars, avantageusement une pression partielle de 5 à 50 bars et en particulier de 10 à 30 bars.

**7.** Procédé selon une ou plusieurs des revendications 1 à 6, caractérisé en ce qu'on introduit pendant la réaction, en continu, du bioxyde de carbone sous pression.

**8.** Procédé selon une ou plusieurs des revendications 1 à 7, caractérisé en ce qu'on fait réagir des composés de formule II, dans lesquels au maximum l'un des restes $R^1$ et $R^2$ représente un groupe alcoxy et avantageusement l'un au moins de ces restes représente un atome d'hydrogène.

**9.** Procédé selon une ou plusieurs des revendications 1 à 8, caractérisé en ce que les restes alkyles contiennent 1 à 4, notamment 1 à 2 atomes de carbone et les restes alcoxy contiennent 1 à 2 atomes de carbone et en particulier ils ne contiennent qu'un seul atome de carbone.

**10.** Procédé selon une ou plusieurs des revendications 1 à 9, caractérisé en ce qu'on sépare par filtration l'urée cyclique du milieu aqueux de réaction et l'on réintroduit dans le procédé le liquide, qui contient éventuellement un catalyseur et des restes de la matière de départ n'ayant pas réagi.